# EUROPEAN PATENT APPLICATION

(11) **EP 3 090 734 A1**
(43) Date of publication of application: **09.11.2016**
(21) Application number: 14877117.3
(22) Date of filing: 30.07.2014
(51) Int. Cl.: A61K 9/48, A61K 47/38, A61K 47/10

(54) **AQUEOUS COMPOSITION FOR HARD CAPSULE, AND HARD CAPSULE PRODUCED USING SAME**

(30) Priority: 31.12.2013 KR 20130168263
(71) Applicant: LOTTE Fine Chemical Co., Ltd., Ulsan, 44714 (KR)
(72) Inventor: SON, Jin Ryul, Incheon 406-736 (KR); CHUN, Jeong Hee, Incheon 401-731 (KR)
(74) Representative: Kador & Partner
(86) International application number: PCT/KR2014/006994
(87) International publication number: WO 2015/102198

(57) **Abstract**

The present invention relates to an aqueous composition for a hard capsule, a method for producing same, and a hard capsule produced using the aqueous composition. More specifically, the present invention relates to an aqueous composition for a hard capsule which comprises a small amount of alcohol and thus has guaranteed stability and excellent capsule moldability, and to a hard capsule produced using the aqueous composition.

## Description

### [Technical Field]

The present invention relates to an aqueous composition for a hard capsule including a water-soluble cellulose ether, a lower alcohol and a glycol, and a hard capsule produced using the same.

### [Background Art]

In general, hard capsules have been produced using gelatin derived from cattle or a pig.

An aqueous composition for a hard capsule including the gelatin has a short production time since the gelatin may be dissolved in water having a high temperature (for example, 60°C). Also, when a mold pin is dipped into the aqueous composition and removed therefrom to dry the aqueous composition coated onto the mold pin, high-quality hard capsules having a short drying time and excellent qualities in elasticity, gloss and disintegrability may be obtained, and the hard capsules may be produced with a very high production yield. However, since the current use of gelatin has been limited due to problems such as bovine spongiform encephalopathy, capsules produced using a plant material such as cellulose ether rather than the gelatin have come into the spotlight.

However, although cellulose ether is soluble in water having a room temperature (25°C), most of the cellulose ether aggregates to form clusters immediately after the cellulose ether is added to water. Therefore, the cellulose ether has a problem in that much time is required to completely dissolve the cellulose ether.

To solve the above problems, one example of a method for preparing an aqueous composition for a hard capsule will be described, as follows.

First of all, cellulose ether is added to water having a high temperature (for example, 80°C or higher) to prevent the occurrence of aggregation, and then thoroughly dispersed to prepare a dispersion. Thereafter, the dispersion is air-cooled to a first temperature (for example, 40 to 50°C) to dissolve the dispersed cellulose ether in water. Subsequently, the resulting solution is warmed to a second temperature (for example, 55 to 65°C), and a gelation agent and an optional gelation auxiliary agent are added to the resulting solution. In this case, a reason for warming the resulting solution to the second temperature is to prevent solidification of the gelation agent and the gelation auxiliary agent.

As conventional technology of producing a hard capsule using the method, Patent Document 1 (Japanese Patent Application Laid-Open Publication No. 2001-245609) discloses a method of producing a cellulose hard capsule using a solution obtained by blending pectin and glycerin with a cellulose base, and a cellulose hard capsule produced by the method, and also discloses that the solubility and transparency of the cellulose capsule may be improved by the method.

However, the cellulose ether is not completely dissolved in water at the second temperature. Therefore, the qualities (elasticity, gloss, disintegrability, etc.) of the capsule are deteriorated due to the presence of insoluble particles, and some problems in a manufacturing process, such as a drop in filtration efficiency in a subsequent filtration process for removing foreign matter, is also caused.

### [Prior-Art Document]

### [Patent Document]

(Patent Document 1) JP2001-245609 A

### [Disclosure]

### [Technical Problem]

Therefore, the present invention is designed to solve the problems of the prior art, and therefore it is an object of the present invention to provide an aqueous composition for a hard capsule which includes a water-soluble cellulose ether and an alcohol.

It is another object of the present invention to provide an aqueous composition for a hard capsule capable of improving solubility of the cellulose ether when the cellulose ether is used together with a lower alcohol as the alcohol, and a glycol.

It is still another object of the present invention to provide a hard capsule produced using the aqueous composition for a hard capsule.

### [Technical Solution]

To solve the problems, according to an aspect of the present invention, there is provided an aqueous composition for a hard capsule which includes a water-soluble cellulose ether, at least one lower alcohol selected from the group consisting of ethanol, methanol, isopropanol, and butanol, and water, wherein a concentration of the lower alcohol is in a range of 1 to 4.9% by weight, based on 100% by weight of the aqueous composition for hard capsule, and the aqueous composition for a hard capsule further includes a glycol at 4 to 50 parts by weight, both exclusive, based on 100 parts by weight of the water-soluble cellulose ether.

An alkylene glycol having 2 to 10 carbon atoms, or a polyalkylene glycol having a weight average molecular weight of 200 to 1,000 may be preferably used as the glycol. More preferably, at least one selected from the group consisting of ethylene glycol, propylene glycol, butylene glycol, pinacol, and a polyethylene glycol having a weight average molecular weight of 400 to 800 may be used.

At least one selected from the group consisting of hydroxypropyl methyl cellulose (HPMC), hydroxyethyl methyl cellulose (HEMC), and methyl cellulose (MC) may be used as the water-soluble cellulose ether.

Also, the aqueous composition for a hard capsule may further includes a gelation agent at 0.05 to 5.0 parts by weight and a gelation auxiliary agent at greater than 0 parts by weight and not more than 1.0 part by weight, both exclusive, based on 100 parts by weight of the aqueous composition for a hard capsule.

According to another aspect of the present invention, there is provided a hard capsule produced using the aqueous composition for a hard capsule.

### [Advantageous Effects]

The aqueous composition for a hard capsule according to one exemplary embodiment of the present invention enables the cellulose ether to be directly dissolved in water having a low temperature (for example, 0 to 40°C) as well as a high temperature (for example, 40 to 70°C). Therefore, a production time of the aqueous composition may be curtailed, thereby improving a process yield of a hard capsule.

Also, stability in a production process can be secured since a small amount of an alcohol is included.

### [Best Mode]

The present invention is directed to an aqueous composition for a hard capsule which includes a water-soluble cellulose ether, at least one lower alcohol selected from the group consisting of ethanol, methanol, isopropanol, and butanol, and water. Here, a concentration of the lower alcohol is in a range of 1 to 4.9% by weight, based on 100% by weight of the aqueous composition for hard capsule, and the aqueous composition for a hard capsule further includes a glycol at 4 to 50 parts by weight, both exclusive, based on 100 parts by weight of the water-soluble cellulose ether.

The alcohol serves to aid in liquefying (that is, dissolving) the water-soluble cellulose ether in the aqueous composition for a hard capsule. Specifically, when the water-soluble cellulose ether is added to water having a low temperature (20 to 30°C), only a portion of the water-soluble cellulose ether coming in direct contact with the water is dissolved, but the other portion which does not come in direct contact with the water aggregates to form clusters. On the other hand, when the water-soluble cellulose ether is added to water having a high temperature (40 to 70°C), even a portion of the water-soluble cellulose ether coming in direct contact with the water tends not to be easily dissolved. However, the alcohol is mixed with water to form an aqueous alcohol solution, and the water-soluble cellulose ether is easily dissolved in an aqueous alcohol solution having a low temperature (20 to 30°C) as well as an aqueous alcohol solution having a high temperature (40 to 70°C).

In the aqueous composition for a hard capsule according to one exemplary embodiment of the present invention, a glycol is used together with a lower alcohol as the alcohol. When the aqueous composition is prepared using only the lower alcohol, the lower alcohol should be included at a concentration of 5% by weight or more, based on the weight of the aqueous composition, and thus solubility of the cellulose ether may be improved. However, when a large amount of the lower alcohol is included, stability issues in a process, such as fire caused by heating in a production process, may be caused.

Accordingly, in the present invention, when a glycol is used together with the lower alcohol, a concentration of the lower alcohol may be minimized, thereby improving solubility of the cellulose ether. That is, according to the present invention, when the concentration of the lower alcohol is in a range of 1 to 4.9% by weight, both exclusive, based on 100% by weight of the aqueous composition, and the concentration of the glycol is in a range of 4 to 50 parts by weight, both exclusive, based on 100% by weight of the water-soluble cellulose ether, the cellulose ether may be dissolved to have no problems in being molded into capsules while ensuring stability.

An alkylene glycol having 2 to 10 carbon atoms, or a polyalkylene glycol having a weight average molecular weight of 200 to 1,000 may be preferably used as the glycol. More preferably, at least one selected from the group consisting of ethylene glycol, propylene glycol, butylene glycol, pinacol, and a having a weight average molecular weight of 400 to 800 may be used.

A concentration of the water-soluble cellulose ether may be in a range of 10 to 25% by weight, based on the weight of the aqueous composition for a hard capsule. When the concentration of the water-soluble cellulose ether is in this range, bubbles may be easily removed since the resin composition may have a proper viscosity, and thus a capsule having a proper thickness may be obtained.

The water-soluble cellulose ether is a main ingredient of the aqueous composition for a hard capsule. Such a water-soluble cellulose ether is derived from cellulose that is a plant material, and thus has an advantage in that it is harmless to humans. In this specification, the term "cellulose ether" refers to a cellulose derivative obtained by etherifying a hydroxyl group of cellulose using an etherifying agent.

At least one selected from the group consisting of hydroxypropyl methyl cellulose (HPMC), hydroxyethyl methyl cellulose (HEMC), and methyl cellulose (MC) may be used as the water-soluble cellulose ether.

Also, the aqueous composition for a hard capsule according to one exemplary embodiment of the present invention may further include a gelation agent and a gelation auxiliary agent. A concentration of the gelation agent may be in a range of 0.05 to 5.0 parts by weight, both exclusive, based on 100 parts by weight of the aqueous composition. Examples of the gelation agent may include at least one gelation agent selected from the group consisting of carrageenan, gellan gum, xanthan gum, and pectin. A concentration of the gelation auxiliary agent may be greater than 0 parts by weight and not more than 1.0 part by weight, both exclusive, based on 100 parts by weight of the aqueous composition. Examples of the gelation auxiliary agent may include at least one gelation auxiliary agent selected from the group consisting of potassium chloride, potassium acetate, and calcium chloride.

Hereinafter, one example of the method for preparing an aqueous composition for a hard capsule will be described in detail.

The method for preparing an aqueous composition for a hard capsule may include mixing water, a lower alcohol and a glycol to prepare an aqueous alcohol solution (S1), heating the aqueous alcohol solution (S2), and adding cellulose ether to the heated aqueous alcohol solution to prepare a cellulose ether solution (S3).

In the step S2, the heating of the mixed solution may be performed at a temperature ranging from room temperature (20 to 30°C) to a temperature of 40 to 70°C. This step S2 is done to readily disperse the water-soluble cellulose ether in the aqueous alcohol solution so that the water-soluble cellulose ether is readily dissolved in the aqueous alcohol solution without aggregation in the step S3. When the heating temperature is in this range, an aqueous composition for a hard capsule having high capsule moldability and exhibiting a minimal increase in energy cost caused by inevitable heating may be obtained without solidifying a gelation agent (and optionally a gelation auxiliary agent) to be described below.

The step S3 may be performed by slowly adding the water-soluble cellulose ether into the heated aqueous alcohol solution while stirring (for example, at 300 rpm).

The method for preparing an aqueous composition for a hard capsule may further include aging the mixed solution of cellulose ether at 40 to 70°C for 2 to 12 hours after the step S3.

When the mixed solution is subjected to an aging process as described above, the water-soluble cellulose ether is completely dissolved, and thus the aqueous composition has the following advantages: first, has a shorter production time; second, exhibits high homogeneity and uniform viscosity, and has no the layor separation of the solution even when stored for a long period of time; third, has a constant viscosity maintained for all production units; fourth, has high capsule moldability since insoluble products (for example, cellulose ether) suppressing the functions of a gelation agent and an optional gelation auxiliary agent do not exist; fifth, has high miscibility between cellulose ether and the gelation agent (and the optional gelation auxiliary agent), resulting in a decrease in the amount of the added gelation agent (and optionally the gelation auxiliary agent); and, sixth, has high filtration efficiency in a subsequent filtration process for removing foreign matter from the aqueous composition for a hard capsule.

In the step S4, a gelation agent or a gelation auxiliary agent may be additionally added to the resulting solution.

At least one of the steps S1 to S4 may be performed while stirring.

The method may further include removing bubbles from the aqueous composition for a hard capsule after the step S4. The step S4 may be performed while stirring.

The functions, types and concentrations of the alcohol, the water-soluble cellulose ether, the gelation agent, and the gelation auxiliary agent are as described above, and thus detailed description thereof are omitted.

However, the method for preparing an aqueous composition for hard capsule according to one exemplary embodiment of the present invention is not limited thereto, and may be widely modified by those skilled in the related art. For example, the steps S1 to S3, which correspond to a process of preparing the cellulose ether solution, may be replaced with the following steps M1 to M3 or step N1.

That is, the cellulose ether solution may be prepared by mixing water, a lower alcohol and a glycol to prepare an aqueous alcohol solution (M1), adding cellulose ether to the aqueous alcohol solution to prepare a cellulose ether solution (M2), and heating the cellulose ether solution to 40 to 70°C (M3).

Also, the cellulose ether solution may be prepared by mixing all of water, a water-soluble cellulose ether, a lower alcohol and a glycol, each of which are heated to 40 to 70°C, to prepare a cellulose ether solution (N1).

Meanwhile, according to the present invention, a hard capsule produced using the aqueous composition for a hard capsule is provided. For example, the hard capsule may be produced by dipping a mold pin kept at room temperature (20 to 30°C) into the aqueous composition for a hard capsule heated to a high temperature (40 to 70°C), removing the mold pin from the aqueous composition, and the drying the aqueous composition on the mold pin (this process is referred to as a 'cold pin process').

Hereinafter, the present invention will be described in further detail with reference to exemplary embodiments thereof. However, it should be understood that the description proposed herein is not intended to limit the scope of the present invention.

### Examples 1 to 4

Ethanol, purified water, polyethylene glycol (PEG), and propylene glycol (PG) were mixed at a ratio as listed in Table 1 to prepare an aqueous alcohol solution. Thereafter, the aqueous alcohol solution was heated to 50°C, and hydroxypropyl methyl cellulose (HPMC) (Samsung Fine Chemicals Co., Ltd., AW4) was added to the aqueous alcohol solution at a ratio as listed in Table 1 to prepare a cellulose ether solution. In this case, a time at which the HPMC was completely dissolved in the aqueous alcohol solution was measured. The measured times are listed in the following Table 1.

### Comparative Example 1

Ethanol and purified water were mixed at a ratio as listed in Table 1 to prepare an aqueous alcohol solution. Thereafter, the aqueous alcohol solution was heated to 50°C, and HPMC (Samsung Fine Chemicals Co., Ltd., AW4) was added to the aqueous alcohol solution at a ratio as listed in Table 1 to prepare a cellulose ether solution. In this case, a time at which the HPMC was completely dissolved in the aqueous alcohol solution was measured. The measured times are listed in the following Table 1.

**[Table 1]**

| | Aqueous composition for a hard capsule (wt%) | | | Glycol (parts by weight)* | | Temperature of aqueous alcohol solution | Dissolution time (hr) |
|---|---|---|---|---|---|---|---|
| | Water | Ethanol | HPMC | PEG | PG | | |
| Example 1 | 76.0 | 4 | 20 | 0 | 1 | 50 | 4 |
| Example 2 | 76.0 | 4 | 20 | 1 | 0 | 50 | 4 |
| Example 3 | 77.0 | 3 | 20 | 0 | 2 | 50 | 5 |
| Example 4 | 77.0 | 3 | 20 | 2 | 0 | 50 | 6 |
| Comparative Example 1 | 76.0 | 4 | 20 | 0 | 0 | 50 | Insoluble |

| | | | | | | | |
|---|---|---|---|---|---|---|---|
| * The concentration of the glycol is represented by part(s) by weight, both exclusive, based on 20 parts by weight of the HPMC. | | | | | | | |

Referring to Table 1 it can be seen that the cellulose ether was completely dissolved within a short period of time in the case of the aqueous alcohol solutions of Examples 1 to 4 in which the glycol, PEG or PG, was used together with a small amount of ethanol according to the present invention. However, it was revealed that the cellulose ether was not completely dissolved in the case of the aqueous alcohol solution of Comparative Example 1 including only the ethanol.

### Experimental Example 1: Evaluation of degree of gelation

0.6 parts by weight (exclusive) of carrageenan, and 0.4 parts by weight (exclusive) of KCl were added to each of the cellulose ether solutions prepared by Examples 1 to 4 and Comparative Example 1, based on 100 parts by weight of the solution, to prepare a test solution for a hard capsule. Thereafter, the total lengths of coating films, which were formed while the respective aqueous compositions flowed down when mold pins (60, 14.5 cm) for testing a degree of gelation at room temperature were dipped into each of the test solutions to a depth of 2.5 cm, removed therefrom and flipped over, are listed in the following Table 2. The degree of gelation was evaluated as the total length of the coating film. Here, the coating film having a shorter total length means that the coating film has a greater degree of gelation.

**[Table 2]**

| | Example 1 | Example 2 | Example 3 | Example 4 | Comparative Example 1 |
|---|---|---|---|---|---|
| Total length (cm) of coating film | 6.5 | 6.4 | 6.8 | 6.7 | Continued flow |

Referring to Table 2, it can be seen that the test solutions of Examples 1 to 4 in which the glycol, PEG or PG, was used together with a small amount of ethanol according to the present invention had a high degree of gelation, and thus exhibited excellent capsule moldability. However, it can be seen that the test solution of Comparative Example 1 including only the ethanol was not easily molded into capsules since the test solution continued to flow down.

## Claims

1. An aqueous composition for a hard capsule comprising a water-soluble cellulose ether; at least one lower alcohol selected from the group consisting of ethanol, methanol, isopropanol, and butanol; and water,
wherein a concentration of the lower alcohol is in a range of 1 to 4.9% by weight, based on 100% by weight of the aqueous composition for hard capsule, and
the aqueous composition for a hard capsule further comprises a glycol at 4 to 50 parts by weight, both exclusive, based on 100 parts by weight of the water-soluble cellulose ether.

2. The aqueous composition for a hard capsule of claim 1, wherein the glycol is an alkylene glycol having 2 to 10 carbon atoms, or a polyalkylene glycol having a weight average molecular weight of 200 to 1,000.

3. The aqueous composition for a hard capsule of claim 1, wherein the glycol comprises at least one selected from the group consisting of ethylene glycol, propylene glycol, butylene glycol, pinacol, and a polyethylene glycol having a weight average molecular weight of 400 to 800.

4. The aqueous composition for a hard capsule of claim 1, wherein the water-soluble cellulose ether comprises at least one selected from the group consisting of hydroxypropyl methyl cellulose (HPMC), hydroxyethyl methyl cellulose (HEMC), and methyl cellulose (MC).

5. The aqueous composition for a hard capsule of claim 1, further comprising a gelation agent at 0.05 to 5.0 parts by weight, both exclusive, based on 100 parts by weight of the aqueous composition for a hard capsule, the gelation agent comprising at least one selected from the group consisting of carrageenan, gellan gum, xanthan gum, and pectin.

6. The aqueous composition for a hard capsule of claim 1, further comprising a gelation auxiliary agent at greater than 0 parts by weight and not more than 1.0 part by weight, both exclusive, based on 100 parts by weight of the aqueous composition for a hard capsule, the gelation auxiliary agent comprising at least one selected from the group consisting of potassium chloride, potassium acetate and calcium chloride.

7. A hard capsule produced using the aqueous composition for a hard capsule defined in any one of claims 1 to 6.
